(19) Europäisches Patentamt / European Patent Office / Office européen des brevets

(11) EP 3 526 229 B1

(12) **EUROPEAN PATENT SPECIFICATION**

(45) Date of publication and mention of the grant of the patent:
**28.04.2021 Bulletin 2021/17**

(21) Application number: **17777935.2**

(22) Date of filing: **09.10.2017**

(51) Int Cl.:
***C07D 309/10*** *(2006.01)*

(86) International application number:
**PCT/EP2017/075664**

(87) International publication number:
**WO 2018/069243 (19.04.2018 Gazette 2018/16)**

(54) **PROCESS FOR PREPARING GLUCOPYRANOSYL-SUBSTITUTED BENZYL-BENZENE DERIVATIVES**

VERFAHREN ZUR HERSTELLUNG VON GLUCOPYRANOSYL-SUBSTITUIERTEN BENZYL-BENZOL-DERIVATEN

PROCÉDÉ DE PRÉPARATION DE DÉRIVÉS DE BENZYL-BENZÈNE À SUBSTITUTION GLUCOPYRANOSYLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.10.2016 EP 16193735**

(43) Date of publication of application:
**21.08.2019 Bulletin 2019/34**

(73) Proprietor: **Boehringer Ingelheim International GmbH**
**55216 Ingelheim am Rhein (DE)**

(72) Inventors:
- **WIRTH, Thomas**
  **55216 Ingelheim Am Rhein (DE)**
- **BERG, Alexander**
  **55216 Ingelheim Am Rhein (DE)**
- **MEYNHARDT, Bernd**
  **55216 Ingelheim Am Rhein (DE)**
- **WEBER, Dirk**
  **55216 Ingelheim Am Rhein (DE)**

(74) Representative: **Simon, Elke Anna Maria et al**
**Boehringer Ingelheim GmbH**
**Binger Strasse 173**
**55216 Ingelheim am Rhein (DE)**

(56) References cited:
**WO-A1-2006/120208 WO-A2-2011/039108**

- **FUERSTNER A ET AL: "Iron-Catalyzed Cross-Coupling Reactions", JOURNAL OF THE AMERICAN CHEMICAL SOCIETY, AMERICAN CHEMICAL SOCIETY, US, vol. 124, 1 January 2002 (2002-01-01), pages 13856-13863, XP002250741, ISSN: 0002-7863, DOI: 10.1021/JA027190T cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

Field of the Invention

The present invention relates to processes for preparing glucopyranosyl-substituted benzyl-benzene derivatives of the formula III,

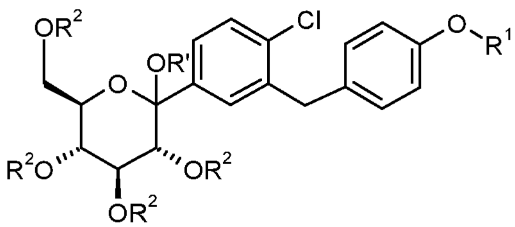

III

wherein the substituents $R^1$, $R^2$ and R' are defined as hereinafter.

In addition, the present invention relates to processes for preparing compounds of the formula IV

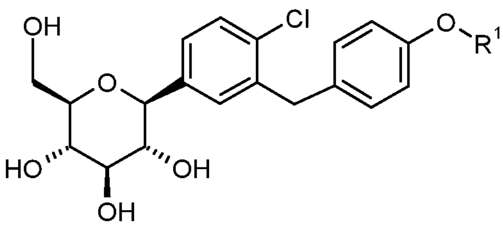

IV

,

which comprise said processes for preparing compounds of formula III, e.g. for the synthesis of inhibitors of the sodium-dependent glucose cotransporter SGLT2.

Background of the Invention

In WO 2005/092877, glucopyranosyl-substituted benzene derivatives of the general formula

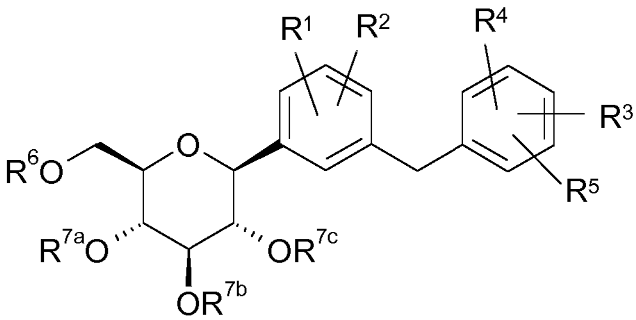

are described wherein the groups $R^1$ to $R^6$ and $R^{7a}$, $R^{7b}$, $R^{7c}$ are as defined therein.

In WO 2006/117359, a crystalline form of 1-chioro-4-(β,-*D*-glucopyranos-1-yl)-2-[4-((*S*)-tetrahydrofuran-3-yloxy)-benzyl]-benzene and its synthesis are described.

In WO 2006/120208, several methods of synthesis of compounds of the general formula

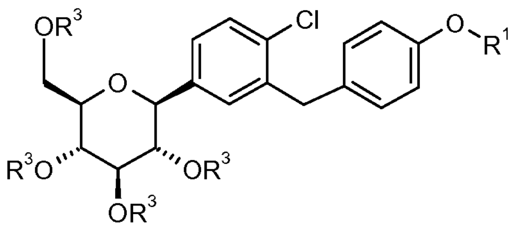

are described wherein $R^1$ denotes, among others, R-tetrahydrofuran-3-yl and S-tetrahydrofuran-3-yl and $R^3$ is as defined therein. The example XVIII therein relates to the synthesis of 1-chloro-4-(β-*D*-glucopyranos-1-yl)-2-(4-(*S*)-tetrahydrofuran-3-yloxy-benzyl)-benzene.

In WO 2011/039108, modified processes are described for preparing glucopyranosyl-substituted benzyl-benzene derivatives of the general formula

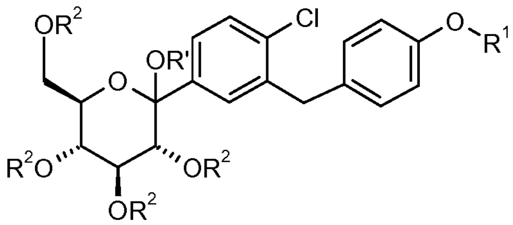

wherein $R^1$ denotes, among others, (R)-tetrahydrofuran-3-yl and (S)-tetrahydrofuran-3-yl and R' and $R^2$ are as defined therein. In these processes, the C-C bond between the glycoside and the aglycone is formed in step (S2) by reaction of a gluconolactone with an organometallic species, for instance an aryl Grignard compound.

It is known, however, that aryl Grignard reagents are prone to homo-coupling reactions, in particular in the presence of transition metal salts. This can be exploited preparatively (Kharasch et al., J. Am. Chem. Soc. 1941, 63, 2316.), but may also be observed as an unwanted side reaction in cross-couplings (Fürstner et al., J. Am. Chem. Soc. 2002, 124, 13856.).

Object of the Invention

The object of the present invention is to provide advantageous processes for preparing a glucopyranosyl-substituted benzyl-benzene derivative of formula III,

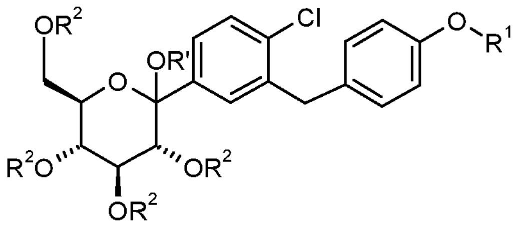

**III**

wherein $R^1$, $R^2$ and R' are defined as hereinafter;
in particular processes conducted under conditions to reduce side reactions that may impact the yield and the impurity profile of the substance obtained by the process.

In particular, an object of the present invention is to provide a process in which unwanted side reactions are reduced by carrying out the process up to and including the C-C bond forming step at sufficiently low concentrations of iron ions, in particular by choosing appropriate qualities of the equipment and purities of the reagents employed.

A further object of the present invention is to provide processes for preparing a compound of formula IV

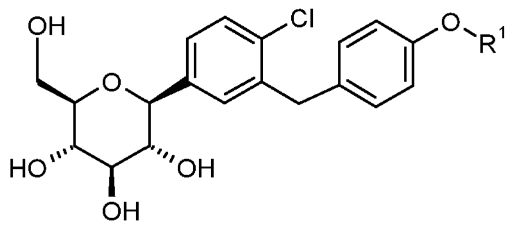

IV

wherein $R^1$ is defined as hereinafter, comprising the above-mentioned processes for preparing compounds of formula III.

Other objects of the present invention will become apparent to the person skilled in the art directly from the foregoing and following description.

Summary of the Invention

In a first aspect, the present invention relates to a process for preparing a glucopyranosyl-substituted benzylbenzene derivative of general formula III,

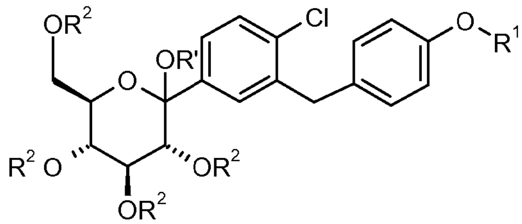

III

wherein

$R^1$ denotes (*R*)-tetrahydrofuran-3-yl or (*S*)-tetrahydrofuran-3-yl; and

$R^2$ independently of one another denote hydrogen, ($C_{1-8}$-alkyl)carbonyl-, ($C_{1-8}$-alkyl)oxycarbonyl-, phenylcarbonyl-, phenyl-($C_{1-3}$-alkyl)-carbonyl-, phenyl-$C_{1-3}$-alkyl-, allyl-, $R^aR^bR^cSi$, $CR^aR^bOR^c$, wherein two adjacent groups $R^2$ may be linked with each other to form a bridging group $SiR^aR^b$, $CR^aR^b$ or $CR^aOR^b$-$CR^aOR^b$; and wherein $R^a$, $R^b$, $R^c$ independently of one another denote $C_{1-4}$-alkyl, phenyl or phenyl-$C_{1-3}$-alkyl-, while the alkyl groups may be mono- or polysubstituted by halogen; while the phenyl groups mentioned in the definition of the above groups may be mono- or polysubstituted with L1, wherein L1 independently of one another are selected from among fluorine, chlorine, bromine, $C_{1-3}$-alkyl, $C_{1-4}$-alkoxy and nitro; and

R' denotes hydrogen, methyl or ethyl;

comprising the steps (S1), (S2) and (S3):

(S1): reacting a compound of general formula I

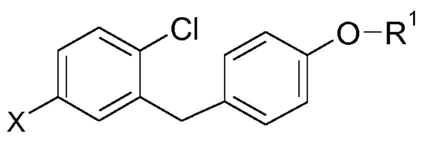

I

wherein $R^1$ is defined as hereinbefore and X denotes Br, I or triflate; with a $C_{1-4}$-alkyl-magnesium chloride or bromide, wherein lithium bromide and/or lithium chloride is optionally used, and

(S2): reacting the organometallic compound obtained in step (S1) with a compound of general formula II

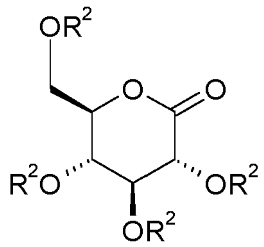

II

wherein $R^2$ is defined as hereinbefore; and
wherein lithium bromide and/or lithium chloride is optionally used, and
wherein $R^2$ not being hydrogen are optionally cleaved during or at the end of (S2), and

(S3): reacting the adduct obtained in step (S2) with a compound R'-OH or a mixture of compounds R'-OH, wherein R' is defined as hereinbefore, in the presence of one or more acids,

characterized in that,
the mole ratio of iron ions in the reaction mixtures of step (S1) and/or step (S2) to compound I employed in step (S1) does not exceed 40 ppm.

In a second aspect, the present invention relates to a process for preparing a compound of general formula IV

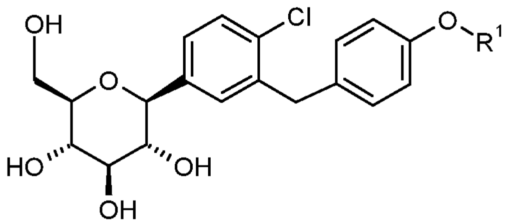

**IV**

wherein $R^1$ is defined as hereinbefore;
comprising said process for preparing a compound of general formula III and further comprising step (S4) and optionally comprising step (S5):

(S4): reacting the compound of general formula III with a reducing agent; and optionally

(S5): cleavage of the protective groups $R^2$ not being hydrogen in the compound formed in step (S4).

Detailed Description of the Invention

In the following, the process steps relevant to this invention are described; they are disclosed in detail in WO 2006/120208 and WO 2011/039108.

Unless otherwise stated, the groups, residues and substituents, particularly $R^1$, $R^2$, $R^a$, $R^b$, $R^c$, R', L1, X, are defined as hereinbefore and hereinafter.

If residues, substituents or groups occur several times in a compound, they may have the same or different meanings.

In the processes according to this invention, the following meanings of groups and substituents are preferred:

$R^1$ preferably denotes (*S*)-tetrahydrofuran-3-yl.
$R^2$ preferably denotes hydrogen, methylcarbonyl, ethylcarbonyl or trimethylsilyl. Most preferably, $R^2$ denotes trimethylsilyl.
$R^a$, $R^b$, $R^c$ independently of one another preferably denote methyl, ethyl, n-propyl, iso-propyl, tert-butyl or phenyl; most preferably methyl.
R' preferably denotes methyl.
X preferably denotes I.

Any and each of the above definitions of the substituents may be combined with one another.

An overview of the reaction steps according to the present invention that lead to the formation of a compound

of general formula III is given in Scheme 1: The glucopyranosyl-substituted benzyl-benzene derivative of formula III may be synthesized by the reaction of D-gluconolactone or a derivative thereof (II) with the desired benzyl-benzene compound in the form of an organometallic compound Ib.

Scheme 1: Overview of the reaction steps for the synthesis of compound III

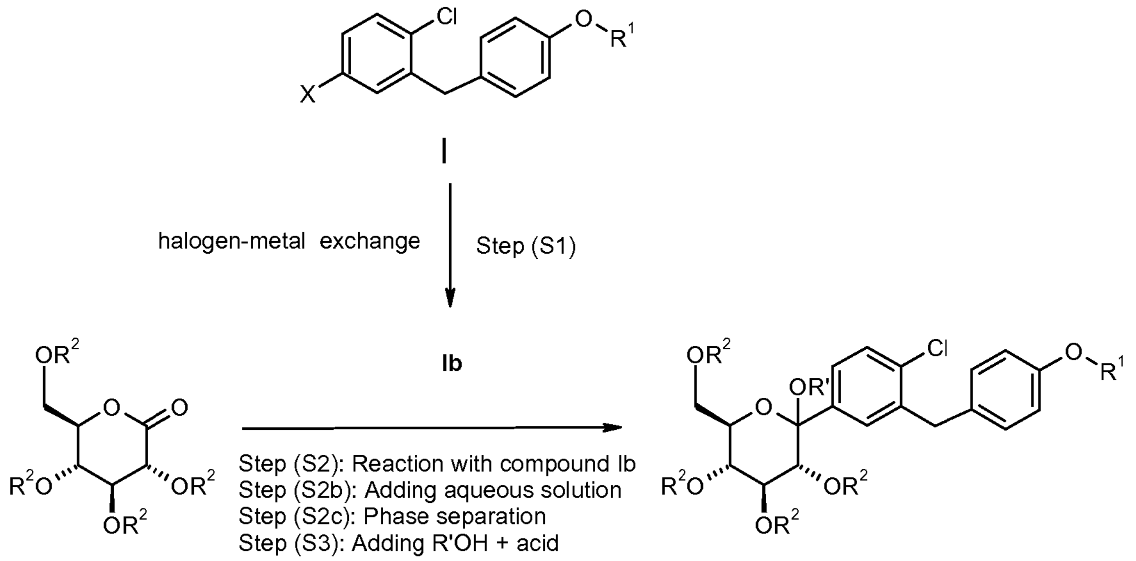


I

II

III

The starting materials for the processes according to the invention, i.e. the compound of formula I and the gluconolactone of formula II, may be synthesized according to the procedures disclosed in WO 2011/039108 (see compounds of formula V and IV, respectively, therein).

The process according to the invention comprises step (S1), a halogen-metal exchange reaction, in which the organometallic compound (Ib) is prepared by reacting the compound of formula I

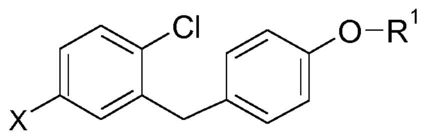

I

with a magnesium Grignard reagent in an organic medium.

The Grignard reagent is preferably a $C_{1-4}$-alkyl-magnesium chloride or bromide, more preferably a $C_{3-4}$-alkyl-magnesium chloride or bromide, most preferably isopropyl magnesium chloride. Optionally, lithium chloride and/or lithium bromide, preferably lithium chloride, may be used, e.g. as promoters, at the beginning of, during or at the end of step (S1). Most preferably, a mixture of isopropyl magnesium chloride and lithium chloride is employed.

In the following, the term "Grignard reagent" shall be used for $C_{1-4}$-alkyl-magnesium chloride and/or bromide, optionally in admixture with lithium chloride and/or bromide. Solutions comprising the Grignard reagent, preferably with tetrahydrofuran (THF), 2-methyltetrahydrofuran or a mixture thereof as the solvent, shall be meant by the term "Grignard solution" (GriS).

Suitable conditions and means (e.g. mole ratios, solvents, further additives, temperatures, reaction times, atmospheric conditions) for carrying out and monitoring the reaction are detailed in WO 2011/039108 or are known to the one skilled in the art.

In particular, the reaction is preferably conducted under the following conditions: The most preferred Grignard reagent is a mixture of isopropyl magnesium chloride and lithium chloride. Most preferably, the Grignard reagent is employed in the form of a solution in tetrahydrofuran. The mole ratio of isopropyl magnesium chloride and lithium chloride is preferably in the range from 1 : 10 to 10 : 1, most preferably about 1 : 1. The most preferred amount of the Grignard reagent relative to the compound of formula I is in range from about 0.5 : 1 to 2 : 1 most preferably about equimolar. Most preferably, the reaction is carried out in THF or 2-methyl-THF or a mixture thereof. The most preferred temperature range is from -40°C to -10°C and the preferred reaction time between 10 min and 600 min. Preferably, the reaction is performed under argon and/or nitrogen inert gas atmosphere. The reaction product of step (S1), the organometallic compound Ib may

be isolated, although such an isolation is not necessary.

In step (S2), the gluconolactone of formula II is added to the organometallic compound Ib in an organic medium, preferably to the reaction mixture obtained in step (S1).

Optionally, lithium chloride and/or lithium bromide, preferably lithium chloride, may be used, e.g. as promoters, at the beginning of, during or at the end of step (S2).

Suitable conditions and means (e.g. mole ratios, solvents, temperatures, reaction times, atmospheric conditions) for carrying out and monitoring the reaction and workup procedures are detailed in WO 2011/039108 or are known to the one skilled in the art.

In particular, the reaction is preferably conducted under the following conditions: Preferably, the reaction is carried out in tetrahydrofuran or 2-methyltetrahydrofurane or a mixture thereof. The preferred amount of the gluconolactone II relative to the organometallic compound Ib is about 1 : 1 to 2 : 1, most preferably about 1.06 : 1. The most preferred temperature range is from -20°C to -5°C and the preferred reaction time between 15 min and 600 min. Preferably, the reaction is performed under argon and/or nitrogen inert gas atmosphere.

The reaction product may be isolated.

In step (S2b), an acidic aqueous solution is added to the reaction mixture obtained in step (S2) such that the reaction mixture forms an aqueous phase and an organic phase whereby the organic phase has a pH in the range from about 0 to 7.

Suitable conditions and means (e.g. acids, acid concentrations, volume ratios, temperatures, addition times, additional salts, additional organic solvents, distillation) for achieving phase separation and measuring the pH value are detailed in WO 2011/039108 or are known to the one skilled in the art.

In particular, the following conditions are preferred: The pH range in the organic phase is preferably from about 1 to 4, most preferably from about 2 to 3. The pH value is measured preferably at a temperature between about 10°C and 30°C. Preferred acids for the aqueous solution are citric acid, acetic acid and tartaric acid, most preferred is citric acid. The acid concentration ranges preferably from 5 to 20 weight-%, most preferably it is about 10 weight-%. The volume of the aqueous solution relative to the volume of the reaction mixture obtained in the step (S2) is most preferably in the range from about 0.3 to 0.6, for example about 0.4. The aqueous solution is added to the reaction mixture most preferably at a temperature from about 10°C to 25°C, most preferably within at least 60 min. Advantageously and most preferably, the volume of the organic phase is reduced by distillation under reduced pressure at a temperature below or equal to about 35°C and further amounts of 2-methyl¬tetrahydrofurane are added, most preferably about 15 to 35 weight-% relative to the total organic phase of the reaction mixture.

Additionally, depending on the nature of $R^2$, cleavage of $R^2$ not being hydrogen may be optionally effected by the reaction conditions applied during step (S2b).

In step (S2c), the organic phase comprising most of the adduct obtained in step (S2) and/or (S2b) is separated from the aqueous phase. The aqueous phase may be washed with an organic medium and the organic phases may be combined. Preferably, the volume of the organic phase is reduced by distillation prior to the next reaction step.

Suitable conditions and means (e.g. solvents, temperature, pressure) for separation of the liquid phases and distillation are detailed in WO 2011/039108 or are known to the one skilled in the art.

In particular, the phase separation is performed most preferably at temperatures from about 0°C to 30°C and the organic solvents are distilled off, preferably under reduced pressure and at temperatures below or equal to 35°C.

In step (S3), the adduct obtained in the preceding steps is reacted with a compound R'-OH or a mixture of compounds R'-OH, wherein R' denotes hydrogen, methyl or ethyl, preferably methyl, in the presence of one or more acids, preferably in the presence of hydrochloric acid. A full conversion to the product of formula III is advantageously achieved by a subsequent distillation. After the completion of the reaction, the remaining acid in the reaction mixture is preferably neutralized by the addition of one or more bases, most preferably triethylamine. A partial or the total amount of the solvent is preferably distilled off.

Suitable conditions and means (e.g. amounts, pH values, temperatures, times, distillation parameters, reduction of water content) for carrying out the reaction and characterizing the product are detailed in WO 2011/039108 or are known to the one skilled in the art.

In particular, the reaction is preferably conducted under the following conditions: The alcohol is preferably employed in an amount exceeding an equimolar amount. By the addition of the one or more acids, a preferred pH value between about 0 and 4, most preferably between 1 and 2 is obtained. The preferred reaction temperature is between 15°C and 25°C and the preferred reaction time is up to 120 min. The distillation is preferably carried out at reduced pressure and at a temperature below or equal about 35°C. The preferred pH range after neutralization is in the range from 5 to 6. Most preferably, the solvent is distilled off at reduced pressure, acetonitrile is added and distilled off again and dichloromethane is added as a solvent.

Additionally, depending on the nature of $R^2$, cleavage of $R^2$ not being hydrogen may optionally be effected by the reaction conditions applied during step (S3).

A compound of formula IV may be synthesized via step (S4), a reduction of the anomeric carbon-oxygen bond

of compound III, and via optional step (S5), the cleavage of $R^2$ not being hydrogen (Scheme 2).

Scheme 2: Reduction and optional deprotection of compound III

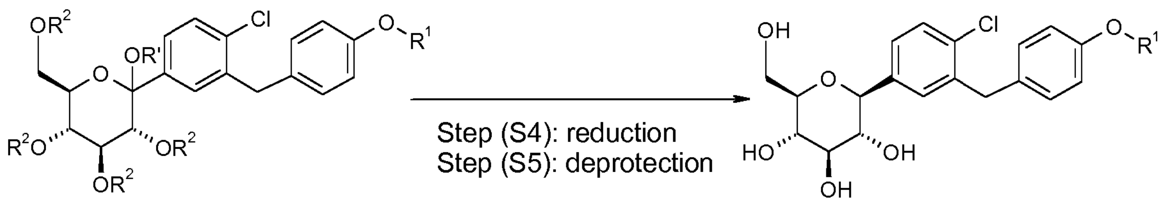


III

IV

$R^1$, $R^2$ and R' are defined as hereinbefore. A preferred meaning of $R^2$ is hydrogen or trimethylsilyl. R' preferably denotes hydrogen, methyl or ethyl, most preferably methyl.

In step (S4), the reduction may be conducted in an organic medium with one or more reducing agents, preferably triethylsilane, in the presence of one or more Lewis acids, preferably aluminium chloride, or without a Lewis acid. Alternatively, in step (S4), hydrogen may be used as reducing agent in the presence of a transition metal catalyst. Suitable conditions and means (e.g. amounts, reducing reagents, Lewis acids, solvents, temperatures, times, atmospheric conditions) for carrying out the reaction and workup procedures are detailed in WO 2011/039108 or are known to the one skilled in the art.

In particular, the reaction is preferably conducted under the following conditions: Preferably the reaction mixture obtained in step (S4) is added to a mixture of one or more organic solvents, the one or more reducing agents and the one or more Lewis acids. The preferred molar amount of the reducing agent relative to compound III is about 2 : 1 to 4 : 1, most preferably about 2.7 : 1. The preferred molar amount of the Lewis acid agent relative to compound III is about 2 : 1 to 4 : 1, most preferably about 2.1 : 1. Most preferred solvents for the reaction are acetonitrile, dichloromethane or mixtures thereof. The preferred reaction temperature is between about 0°C and 30°C, most preferably between 10°C and 20°C. The reaction components are added preferably within 45 min to 120 min and the mixture is preferably stirred for about 30 min to 120 min at about 0°C to 35°C, most preferably at about 15°C to 25°C. Preferably, the reaction is performed under argon and/or nitrogen inert gas atmosphere.

Additionally, depending on the nature of $R^2$, cleavage of $R^2$ not being hydrogen may optionally be effected by the reaction conditions applied during step (S4).

In an optional step (S5), the protective groups $R^2$ not being hydrogen are cleaved from the compound obtained in step (S4), resulting in the compound of formula IV.

Suitable conditions for achieving this depend on the nature of $R^2$, but are detailed in WO 2011/039108 or are known to the one skilled in the art.

The product may be obtained by crystallisation, for example as described in WO 2006/117359 or WO 2011/039108.

It was found that the performance of this process is particularly sensitive to the presence of iron ions, in particular in steps (S1) and (S2): With increasing iron ion concentrations, the formation of oligomers of I and the like was observed so that the yield and the impurity profile of the obtained product are impaired.

This effect was demonstrated experimentally by adding different levels of iron ions to Grignard solutions (isopropyl magnesium chloride and lithium chloride in tetrahydrofuran) to be used in the process according to the invention. This was performed either via direct spiking of iron salts (in order to simulate iron ion impurities present in the reaction mixtures) or by adding pre-treated metal test pieces (in order to simulate the release of iron ions from reactor materials into the solution).

The amount of iron ions was investigated by means of ICP-MS. At the end of step (S1), the amount of oligomers formed was determined via HPLC-UV. At the end of step (S2), the amount of the actually desired hemiacetal product (compound of formula III wherein R' denotes H) was measured by HPLC-UV. The results of these investigations are summarized in the section "Description and Results of Experimental Procedures".

The spiking experiments revealed that even iron ion mass fractions (e.g. $Fe^{2+}$ and/or $Fe^{3+}$) in the low single-digit ppm range in the Grignard solution promote the formation of oligomers of I and the like to a substantial degree and largely suppress the formation of the desired hemiacetal intermediate.

Thus, according to one embodiment of the present invention, the mole ratio of iron ions in the reaction mixtures of step (S1) and/or (S2) to compound I employed in step (S1) does not exceed 40 ppm, preferably 30 ppm, most preferably 20 ppm.

According to another embodiment of the present invention, the mole ratio of iron ions in the reaction mixtures of steps (S1) and/or (S2) to alkyl-magnesium species employed in step (S1) does not exceed 40 ppm, preferably 30 ppm, most preferably 20 ppm.
According to another embodiment of the present invention, the mole ratio of iron ions in the reaction mixture of step (S2) to compound II employed in step (S2) does not exceed 40 ppm, preferably 30 ppm, most preferably 20 ppm.
According to another embodiment of the present invention, the mass fraction of iron ions in the reaction mixtures of steps (S1) and/or (S2) does not exceed 1.5 ppm, preferably 1.1 ppm, most preferably 0.75 ppm.

As a consequence, reagents, in particular Grignard solutions, with very low iron ion concentrations are advantageously employed in the process of the invention.
Thus, according to one embodiment of the present invention, the mole ratio of iron ions in the Grignard solution to $C_{1-4}$-alkyl-magnesium species in the Grignard solution does not exceed 40 ppm, preferably 30 ppm, most preferably 20 ppm.
According to another embodiment of the present invention, the mass fraction of iron ions in the Grignard solution employed in step (S1) does not exceed 3 ppm, preferably 2.2 ppm, most preferably 1.5 ppm.

As a further potential source of iron ions, different reactor materials were tested for the process of the invention (see section "Description and Results of Experimental Procedures"); they were in fact found to be able to release iron ions to different extents when corrosion or oxidation processes were simulated by pre-treatment of the metal test pieces. Such corrosion or oxidation processes are common and well known events in dedicated or multi-purpose chemical manufacturing equipment (e.g. reactors, tubing, containers etc.) and may be induced or accelerated by corrosive agents (e.g. hydrochloric acid) and the presence of oxygen. Corrosive agents (e.g. hydrochloric acid) and oxygen are abundant in any dedicated or multi-purpose chemical manufacturing plant. Another factor influencing these corrosion processes is the type or quality of the construction materials used for the reactors, tubing and containers. The above described corrosion processes can lead to leaching of iron ions into the reaction mixtures of steps (S1) and/or (S2), as defined hereinbefore, resulting in iron ion mass fractions above 0.75 ppm and the formation of oligomers of I.

Therefore, according to another embodiment of the present invention, the process of the invention is carried out in equipment in which the materials of the surfaces that may come into contact with the Grignard solution and/or with the reaction mixtures of steps (S1) and/or (S2), in particular the materials of those surfaces that are in contact with the reaction mixtures during the performance of the reactions, are resistant against releasing or leaching of iron ions into the reaction mixtures under the reaction conditions of steps (S1) and/or (S2) described hereinbefore and hereinafter.
The above-mentioned resistance to releasing or leaching of iron ions shall mean that the above-mentioned criteria for mass fractions and mole ratios of iron ions in the Grignard solution and in the reaction mixtures of steps (S1) and/or (S2) are met.
Thus, preferably, the materials of said surfaces are selected from the group consisting of metal alloys, in particular nickel alloys, with iron mass fractions of not more than 10%, preferably of not more than 6%, most preferably of not more than 1.5%. Non-limiting examples of such metal alloys are Alloy 22 (2.4602) with a typical Fe mass fraction of up to 6% and Alloy 59 (2.4605) with a typical Fe mass fraction of up to 1.5%.
According to another embodiment of the invention, the materials of said surfaces are selected from the group consisting of materials that are treated and/or coated to prevent releasing or leaching of iron ions. Non-limiting examples are glass-lined, metal-plated or polymer-coated surfaces, e.g. glass-lined steel.

**Description and Results of Experimental Procedures:**

**Experiment A**

Pre-Treatment of Grignard solution (GriS; *i*-PrMgCl/ LiCl in THF):

In a glass flask, to a 1.3 mol/L solution of *i*-PrMgCl/ LiCl in THF (100 mL) the respective iron salt ($FeI_2$ or $FeCl_3$) was spiked and the resulting mixture was stirred at room temperature for 7 days under argon atmosphere. Then, a sample was taken and analyzed for the iron ion content with analytical method A.

Description of the Experiment:

In a glass flask, a solution of compound I, wherein X denotes I and $R^1$ denotes (*S*)-tetrahydrofuran-3-yl, (0.072 mol) in THF (54 mL) was cooled to -15°C to -40 °C under argon atmosphere. 55 mL of the pre-treated Grignard solution (1.0 eq) were added at -15°C to -40 °C within 60-65 min. A sample was taken and analyzed for compound I and oligomers with analytical method B and C, respectively. To this solution, compound II, wherein $R^2$ denotes trimethylsilyl, (1.1 eq) was added at -5°C to -25 °C. After completion of the addition, the resulting mixture was stirred at -5°C to -15°C for additional 60-120 min. A sample was taken and analyzed for the hemiacetal intermediate of formula III (R' = H) with

analytical method D.

**Table 1: Results of Experiment A**

| Spiked iron salt | Mass fraction w(Fe) in GriS [ppm] (method A) | Mole ratio r (Fe/Mg) [ppm][2] | Amount of unreacted I [area%] (method B) | Amount of oligomers of I [area%] (method C) | Amount of hemiacetal III [area%] (method D) |
|---|---|---|---|---|---|
| no spiking[1] | *not applicable* | *not applicable* | 3.9 | 0.7 | 82.0 |
| $FeI_2$ | 4.0 | 54 | 10.2 | 60.8 | 1.8 |
| $FeCl_3$ | 10.0 | 135 | 46.1 | 70.3 | *not detected* |
| [1] reference experiment<br>[2] calculated from mass fraction w(Fe) in Grignard solution (see analytical method A) | | | | | |

**Experiment B**

Pre-Treatment of the metal test piece:

The respective metal test piece was stored in a desiccator under an atmosphere of 5M aqueous hydrochloric acid for 4 weeks.

Pre-Treatment of Grignard solution (GriS; *i*-PrMgCl/ LiCl in THF):

In a glass flask, to a 1.3 mol/L solution of *i*-PrMgCl/ LiCl in THF (100 mL) the respective pre-treated metal test piece was added and the resulting mixture was stirred at room temperature for 7 days under argon atmosphere. Then, a sample was taken and analyzed for the iron ion content with analytical method A.

Description of the Experiment:

In a glass flask, a solution of compound I, wherein X denotes I and $R^1$ denotes (*S*)-tetrahydrofuran-3-yl, (0.072 mol) in THF (54 mL) was cooled to -15 °C to -40 °C under argon atmosphere. 55 mL of the pre-treated Grignard solution (1.0 eq) was added at -15 °C to -40 °C within 60-65 min. A sample was taken and analyzed for compound I and oligomers with analytical method B and C, respectively. To this solution, compound II, wherein $R^2$ denotes trimethylsilyl, (1.1 eq) was added at -5 °C to -25 °C. After completion of the addition, the resulting mixture was stirred at -5 °C to -15 °C for additional 60-120 min. A sample was taken and analyzed for the hemiacetal intermediate of formula III (R' = H) with analytical method D.

**Table 2: Results of Experiment B**

| Spiked metal test piece | Mass fraction w (Fe) in GriS [ppm] (method A) | Mole ratio r (Fe/Mg) [ppm][2] | Amount of unreacted I [area%] (method B) | Amount of oligomers of I [area%] (method C) | Amount of hemiacetal III [area%] (method D) |
|---|---|---|---|---|---|
| no spiking[1] | *not applicable* | *not applicable* | 3.9 | 0.7 | 82.0 |
| Alloy 59 (2.4605) | < 1.5 | < 20 | 7.2 | 23.5 | 67.8 |
| Stainless steel A4L (1.4404) | 19 | 256 | 71.3 | 50.4 | *not detected* |
| Stainless steel V2A (1.4301) | 15 | 202 | 68.1 | 59.4 | *not detected* |

(continued)

| Spiked metal test piece | Mass fraction w (Fe) in GriS [ppm] (method A) | Mole ratio r (Fe/Mg) [ppm][2] | Amount of unreacted I [area%] (method B) | Amount of oligomers of I [area%] (method C) | Amount of hemiacetal III [area%] (method D) |
|---|---|---|---|---|---|
| flat steel (P265GH) | 228 | 3078 | 81.6 | 28.3 | *not detected* |
| [1] reference experiment<br>[2] calculated from mass fraction w(Fe) in Grignard solution (see analytical method A) | | | | | |

**Description of Analytical Methods:**

**Analytical Method A**

For the quantification of iron ion concentrations, a quantitative analytical method using ICP-MS (e.g. Perkin Elmer Nexion 300) was used. Samples were filtered using membrane filters (e.g. Pall Acrodisc Premium 25 mm Syringe Filter 0.45 μm GHP Membrane) and were, after addition of nitric acid and hydrochloric acid, digested using a microwave (e.g. Anton Paar Multiwave 3000). Iron ion amounts in solution are determined as mass fractions w(Fe), i.e. the mass of iron ions divided by the mass of the solution, and are given in this document as ppm, i.e. μg (Fe) / g (solution).

The mass fraction of iron ions in the Grignard solution (w(Fe)) may be converted into the mole ratio of iron ions to organomagnesium species (r(Fe/Mg), i.e. the molar amount of iron ions divided by the molar amount of organomagnesium species) with the help of the following formula:

$$r\left(\frac{Fe}{Mg}\right) = \frac{n(Fe)}{n(Mg)} = \frac{w(Fe)}{c(Mg)} * \frac{\rho(GriS)}{M(Fe)}$$

wherein p(GriS) means the density of the Grignard solution (980 g/L), c(Mg) the molar concentration of the Grignard solution (1.3 mol/L) and M(Fe) the molar mass of iron (55.845 g/mol). The mole ratios are given in ppm, i.e. μmol (Fe) / mol (Mg).

**Analytical Method B**

Reaction monitoring method: Gradient HPLC apparatus; eluent A: 1.0 mL trifluoroacetic acid dissolved in 1.0 L HPLC water; eluent B: 1.0 mL trifluoroacetic acid dissolved in 1.0 L gradient grade acetonitrile; HPLC column: Agilent, Zorbax Eclipse XDB-C8, 4.6*150 mm, particle size 5 μm; column temperature: 25 °C; flow: 2.0 mL/min; gradient profile: 0 min, 30% eluent A, 70% eluent B; 5 min, 20% eluent A, 80% eluent B; equilibration 5 min; sample preparation: direct quench of 0.1 mL reaction mixture with 10 mL methanol; injection volume: 1.0 μL; UV-detection: 230 nm; data evaluation: only peaks of compound I (X = I, $R^1$ = (S)-tetrahydrofuran-3-yl; retention time approx. 3.2 min) and quenched intermediate (compound I with X = H, $R^1$ = (S)-tetrahydrofuran-3-yl;, retention time approx. 2.2 min) are taken into account for area% calculation.

**Analytical Method C**

Oligomer monitoring method: Gradient HPLC apparatus; eluent A: 1.0 mL perchloric acid dissolved in 1.0 L HPLC water; eluent B: gradient grade acetonitrile; column: AMT Halo C8, 4.6*150 mm, particle size 2.7 μm; column temperature: 35 °C; flow: 1.5 mL/min; gradient profile: 0 min, 60% eluent A, 40% eluent B; 20 min, 10% eluent A, 90% eluent B; 25 min, 0% eluent A, 100% eluent B; 35 min, 0% eluent A, 100% eluent B; equilibration 5 min; sample preparation: direct quench of 0.1 mL reaction mixture with 10 mL methanol; dilute 500 μL of quenched solution with 500 μL THF; injection volume: 1.0 μL; UV-detection: 224 nm; data evaluation: all peaks in chromatogram are taken into account for area% calculation, peaks eluting later than compound I (X = I, $R^1$ = (S)-tetrahydrofuran-3-yl; retention time approx. 11.4 min) are summarized and reported as "oligomers of compound I".

**Analytical Method D**

Reaction monitoring method: Gradient HPLC apparatus; eluent A: 1.0 mL trifluoroacetic acid dissolved in 1.0 L HPLC water; eluent B: 1.0 mL trifluoroacetic acid dissolved in 1.0 L gradient grade acetonitrile; HPLC column: Agilent, Zorbax Eclipse XDB-C8, 4.6*150 mm, particle size 5 μm; column temperature: 25 °C; flow: 1.2 mL/min; gradient profile: 0 min, 70% eluent A, 30% eluent B; 7 min, 60% eluent A, 40% eluent B; 15 min, 5% eluent A, 95% eluent B; 30 min, 5% eluent A, 95% eluent B; equilibration 7 min; sample preparation: direct quench of 0.1 mL reaction mixture with 5 mL 1 N hydrochloric acid, dilute with 5 mL acetonitrile; injection volume: 1.0 μL; UV-detection: 230 nm; data evaluation: all peaks integrated for area% calculation; reported hemiacetal intermediate (compound of formula III wherein R' = H, $R^1$ = (S)-tetrahydrofuran-3-yl, $R^2$ = trimethylsilyl) at retention time approx. 3.9 min.

## Claims

1. Process for preparing a compound of general formula III,

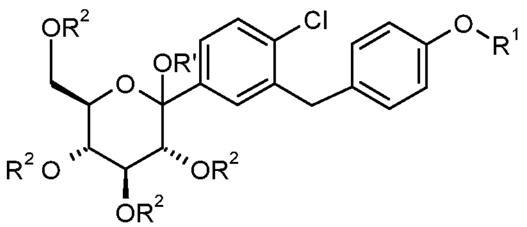

III

wherein $R^1$ denotes (*R*)-tetrahydrofuran-3-yl or (*S*)-tetrahydrofuran-3-yl; and
wherein $R^2$ independently of one another denote hydrogen, ($C_{1-8}$-alkyl)carbonyl, ($C_{1-8}$-alkyl)oxycarbonyl, phenylcarbonyl, phenyl-($C_{1-3}$-alkyl)-carbonyl, phenyl-$C_{1-3}$-alkyl, allyl, $R^aR^bR^cSi$, $CR^aR^bOR^c$, wherein two adjacent groups $R^2$ may be linked with each other to form a bridging group $SiR^aR^b$, $CR^aR^b$ or $CR^aOR^b$-$CR^aOR^b$;
wherein $R^a$, $R^b$, $R^c$ independently of one another denote $C_{1-4}$-alkyl, phenyl or phenyl-$C_{1-3}$-alkyl, while the alkyl groups may be mono- or polysubstituted by halogen;
while the phenyl groups mentioned in the definition of the above groups may be mono- or polysubstituted with L1, wherein L1 independently of one another are selected from among fluorine, chlorine, bromine, $C_{1-3}$-alkyl, $C_{1-4}$-alkoxy and nitro; and
wherein R' denotes hydrogen, methyl or ethyl;

comprising the steps (S1), (S2) and (S3):

(S1): reacting a compound of general formula I

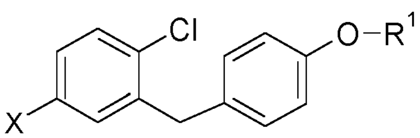

I

wherein $R^1$ is defined as hereinbefore and X denotes Br, I or triflate;
with a $C_{1-4}$-alkyl-magnesium chloride or bromide,
wherein lithium bromide and/or lithium chloride is optionally used, and
(S2): reacting the organometallic compound obtained in step (S1) with a compound of general formula II

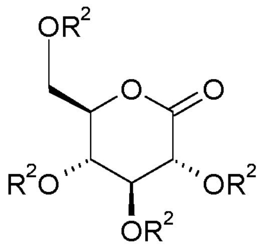

II

wherein $R^2$ is defined as hereinbefore; and
wherein lithium bromide and/or lithium chloride is optionally used, and
wherein $R^2$ not being hydrogen are optionally cleaved during or at the end of (S2), and
(S3): reacting the adduct obtained in step (S2) with a compound R'-OH or a mixture of compounds R'-OH, wherein R' is defined as hereinbefore, in the presence of one or more acids,

**characterized in that**,
the mole ratio of iron ions in the reaction mixtures of step (S1) and/or (S2) to compound I employed in step (S1) does not exceed 40 ppm.

2. The process according to claim 1 wherein X in step (S1) denotes I.

3. The process according to any of claims 1 and 2 wherein $C_{3-4}$-alkyl-magnesium chloride or bromide, preferably isopropyl magnesium chloride is employed in step (S1) and additionally lithium chloride is used in step (S1).

4. The process according to any of claims 1 to 3 wherein $R^2$ denotes trimethylsilyl in the compound of general formula II used in step (S2).

5. The process according to any of claims 1 to 4 wherein R' in step (S3) denotes methyl.

6. Process for preparing a compound of general formula IV

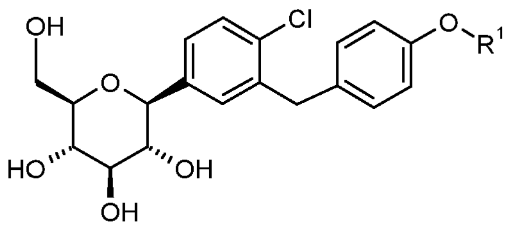

IV

wherein $R^1$ is defined as hereinbefore;
comprising the process according to any of claims 1 to 5 and further comprising step (S4) and optionally comprising step (S5):

(S4): reacting the compound of general formula III with a reducing agent; and optionally
(S5): cleavage of the protective groups $R^2$ not being hydrogen in the compound formed from the compound of general formula III in step (S4).

7. The process according to any of claims 1 to 6 wherein, in the reagent comprising the alkyl-magnesium species used in step (S1) and/or in a solution comprising such reagent, the mole ratio of iron ions to $C_{1-4}$-alkyl-magnesium species does not exceed 40 ppm.

8. The process according to any of claims 1 to 7 wherein the materials of the equipment surfaces that may come into contact with a solution comprising the alkyl-magnesium species used in step (S1) and/or with the reaction mixtures of steps (S1) and/or (S2) are resistant against releasing or leaching of iron ions such that the mole ratio of iron ions in the reaction mixtures of step (S1) and/or (S2) to compound I employed in step (S1) does not exceed 40 ppm.

9. The process according to any of claims 1 to 8 wherein the materials of the equipment surfaces that may come into

contact with a solution comprising the alkyl-magnesium species used in step (S1) are resistant against releasing or leaching of iron ions such that, in said solution, the mole ratio of iron ions to $C_{1-4}$-alkyl-magnesium species does not exceed 40 ppm.

**10.** The process according to any of claims 1 to 9 wherein the materials of the equipment surfaces that may come into contact with a solution comprising the alkyl-magnesium species used in step (S1) and/or with the reaction mixtures of steps (S1) and/or (S2) are selected from the group consisting of metal alloys with iron mass fractions of not more than 10%.

**11.** The process according to any of claims 1 to 10 wherein the materials of the equipment surfaces that may come into contact with a solution comprising the alkyl-magnesium species used in step (S1) and/or the reaction mixtures of steps (S1) and/or (S2) are selected from the group consisting of materials that are treated and/or coated to prevent releasing or leaching of iron ions.

## Patentansprüche

**1.** Verfahren zur Herstellung einer Verbindung der allgemeinen Formel III,

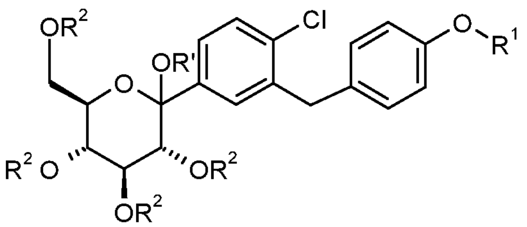

III

wobei $R^1$ (*R*)-Tetrahydrofuran-3-yl oder (*S*)-Tetrahydrofuran-3-yl bezeichnet; und
wobei $R^2$ unabhängig voneinander Wasserstoff, ($C_{1-8}$-Alkyl)carbonyl, ($C_{1-8}$-Alkyl)-oxycarbonyl, Phenylcarbonyl, Phenyl-($C_{1-3}$-alkyl)-carbonyl, Phenyl-$C_{1-3}$-alkyl, Allyl, $R^aR^bR^cSi$, $CR^aR^bOR^c$ bezeichnet, wobei zwei benachbarte Gruppen $R^2$ miteinander verbunden sein können, um eine Brückengruppe $SiR^aR^b$, $CR^aR^b$ oder $CR^a$-$OR^b$-$CR^aOR^b$ zu bilden;
wobei $R^a$, $R^b$, $R^c$ unabhängig voneinander $C_{1-4}$-Alkyl, Phenyl oder Phenyl-$C_{1-3}$-alkyl bezeichnen, wobei die Alkylgruppen mit Halogen mono- oder polysubstituiert sein können;
wobei die in der Definition der obigen Gruppen genannten Phenylgruppen mit L1 mono- oder polysubstituiert sein können, wobei L1 unabhängig voneinander ausgewählt ist aus Fluor, Chlor, Brom, $C_{1-3}$-Alkyl, $C_{1-4}$-Alkoxy und Nitro; und
wobei R' Wasserstoff, Methyl oder Ethyl bezeichnet;

umfassend die Schritte (S1), (S2) und (S3):

(S1): Umsetzen einer Verbindung der allgemeinen Formel I

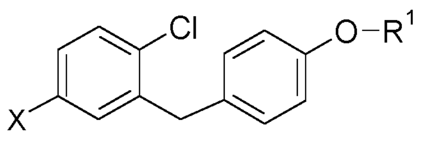

I

,

wobei $R^1$ wie hier zuvor definiert ist und X Br, I oder Triflat bezeichnet;
mit einem $C_{1-4}$-Alkyl-Magnesiumchlorid oder -bromid,
wobei gegebenenfalls Lithiumbromid und/oder Lithiumchlorid verwendet wird, und
(S2): Umsetzen der in Schritt (S1) erhaltenen organometallischen Verbindung mit einer Verbindung der allgemeinen Formel II

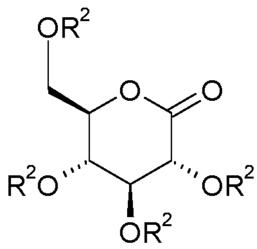

II

,

wobei $R^2$ wie hier zuvor definiert ist; und
wobei gegebenenfalls Lithiumbromid und/oder Lithiumchlorid verwendet wird, und
wobei $R^2$, wenn es nicht Wasserstoff ist, gegebenenfalls während oder am Ende von (S2) abgespalten wird, und
(S3): Umsetzen des in Schritt (S2) erhaltenen Addukts mit einer Verbindung R'-OH oder einer Mischung von Verbindungen R'-OH, wobei R' wie hier zuvor definiert ist, in Gegenwart von einer oder mehreren Säuren,

**dadurch gekennzeichnet, dass**
das Molverhältnis der Eisenionen in den Reaktionsmischungen der Schritte (S1) und/oder (S2) zur in Schritt (S1) eingesetzten Verbindung I 40 ppm nicht übersteigt.

**2.** Verfahren nach Anspruch 1, wobei X in Schritt (S1) I bezeichnet.

**3.** Verfahren nach irgendeinem der Ansprüche 1 und 2, wobei $C_{3-4}$-Alkyl-Magnesiumchlorid oder -bromid, bevorzugt Isopropylmagnesiumchlorid, in Schritt (S1) verwendet wird und zusätzlich Lithiumchlorid in Schritt (S1) verwendet wird.

**4.** Verfahren nach irgendeinem der Ansprüche 1 bis 3, wobei $R^2$ in der in Schritt (S2) eingesetzten Verbindung der allgemeinen Formel II Trimethylsilyl bezeichnet.

**5.** Verfahren nach irgendeinem der Ansprüche 1 bis 4, wobei R' in Schritt (S3) Methyl bezeichnet.

**6.** Verfahren zur Herstellung einer Verbindung der allgemeinen Formel IV

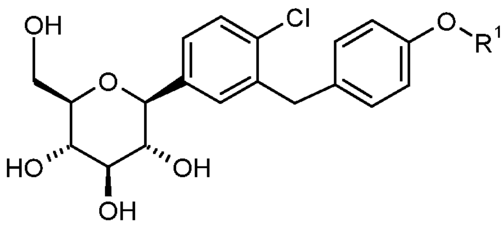

IV

,

wobei $R^1$ wie hier zuvor definiert ist;
umfassend das Verfahren nach irgendeinem der Ansprüche 1 bis 5, und weiterhin umfassend Schritt (S4) und gegebenenfalls umfassend Schritt (S5):

(S4): Umsetzen der Verbindung der allgemeinen Formel III mit einem Reduktionsmittel; und gegebenenfalls
(S5): Abspalten der Schutzgruppen $R^2$, die in der aus der Verbindung der allgemeinen Formel III in Schritt (S4) gebildeten Verbindung nicht Wasserstoff sind.

**7.** Verfahren nach irgendeinem der Ansprüche 1 bis 6, wobei in dem Reagenz, das die Alkyl-Magnesium-Spezies umfasst, die in Schritt (S1) verwendet wird, und/oder in einer Lösung, umfassend ein solches Reagenz, das Molverhältnis von Eisenionen zu der $C_{1-4}$-Alkyl-Magnesium-Spezies 40 ppm nicht übersteigt.

**8.** Verfahren nach irgendeinem der Ansprüche 1 bis 7, wobei die Materialien der Geräteoberflächen, die mit einer Lösung, umfassend die in Schritt (S1) verwendete Alkyl-Magnesium-Spezies, und/oder mit den Reaktionsmischungen der Schritte (S1) und/oder (S2) in Kontakt kommen können, gegen die Freisetzung oder Auslaugung von Eisenionen resistent sind, so dass das Molverhältnis von Eisenionen in den Reaktionsmischungen der Schritte (S1)

und/oder (S2) zur in Schritt (S1) eingesetzten Verbindung I 40 ppm nicht übersteigt.

**9.** Verfahren nach irgendeinem der Ansprüche 1 bis 8, wobei die Materialien der Geräteoberflächen, die mit einer Lösung, umfassend die in Schritt (S1) verwendete Alkyl-Magnesium-Spezies, in Kontakt kommen können, gegen die Freisetzung oder Auslaugung von Eisenionen resistent sind, so dass in der Lösung das Molverhältnis von Eisenionen zu der $C_{1-4}$-Alkyl-Magnesium-Spezies 40 ppm nicht übersteigt.

**10.** Verfahren nach irgendeinem der Ansprüche 1 bis 9, wobei die Materialien der Geräteoberflächen, die mit einer Lösung, umfassend die in Schritt (S1) verwendete Alkyl-Magnesium-Spezies, und/oder mit den Reaktionsmischungen der Schritte (S1) und/oder (S2) in Kontakt kommen können, ausgewählt sind aus der Gruppe, bestehend aus Metalllegierungen mit einem Eisenmassenanteil von nicht mehr als 10 %.

**11.** Verfahren nach irgendeinem der Ansprüche 1 bis 10, wobei die Materialien der Geräteoberflächen, die mit einer Lösung, umfassend die in Schritt (S1) verwendete Alkyl-Magnesium-Spezies, und/oder mit den Reaktionsmischungen der Schritte (S1) und/oder (S2) in Kontakt kommen können, ausgewählt sind aus der Gruppe, bestehend aus Materialien, die behandelt und/oder beschichtet sind, um die Freisetzung oder Auslaugung von Eisenionen zu verhindern.

## Revendications

**1.** Procédé de préparation d'un composé de formule générale III,

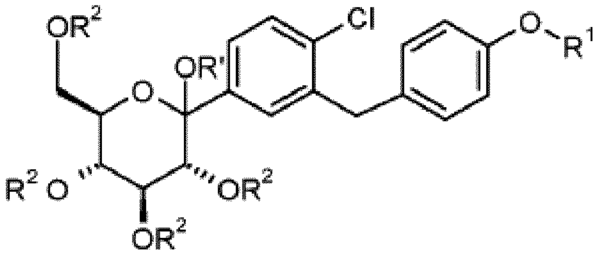

III

dans lequel $R^1$ indique un (R)-tétrahydrofuran-3-yle ou un (S)-tétrahydrofuran-3-yle ; et
dans lequel $R^2$, indépendamment les uns des autres, indiquent un hydrogène, un ($C_{1-8}$alkyl)-carbonyle, un ($C_{1-8}$-alkyl)oxycarbonyle, un phényl-carbonyle, un phényl-($C_{1-3}$-alkyl)-carbonyle, un phényl-$C_{1-3}$-alkyle, un allyle, $R^aR^bR^cSi$, $CR^aR^bOR^c$, dans lequel deux groupes $R^2$ adjacents peuvent être liés l'un à l'autre pour former un groupe pont $SiR^aR^b$, $CR^aR^b$ ou $CR^aOR^b$-$CR^aOR^b$ ;
dans lequel $R^a$, $R^b$, $R^c$, indépendamment les uns des autres, indiquent un $C_{1-4}$-alkyle, un phényle ou un phényl-$C_{1-3}$-alkyle, tandis que les groupes alkyle peuvent être mono- ou polysubstitués avec un halogène ;
tandis que les groupes phényle mentionnés dans la définition des groupes ci-dessus peuvent être mono- ou polysubstitués avec L1, dans lequel L1, indépendamment les uns des autres, sont sélectionnés parmi le fluor, le chlore, le brome, un $C_{1-3}$-alkyle, un $C_{1-4}$-alcoxy et un nitro ; et
dans lequel R' indique un hydrogène, un méthyle ou un éthyle ;

comprenant les étapes (S1), (S2) et (S3) :

(S1) : la réaction d'un composé de formule générale I

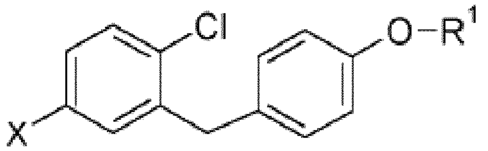

I

dans lequel $R^1$ est défini comme ci-dessus et X indique Br, I ou un triflate ;
avec un chlorure ou bromure de $C_{1-4}$-alkyl-magnésium,
dans lequel du bromure de lithium et/ou du chlorure de lithium sont facultativement utilisés, et

(S2) : la réaction du composé organométallique obtenu dans l'étape (S1) avec un composé de formule générale II

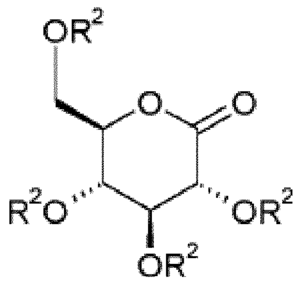

II

dans lequel $R^2$ est défini comme ci-dessus ; et
dans lequel du bromure de lithium et/ou du chlorure de lithium sont facultativement utilisés, et
dans lequel les $R^2$ qui ne sont pas un hydrogène sont facultativement clivés pendant ou à la fin de (S2), et
(S3) : la réaction de l'adduit obtenu dans l'étape (S2) avec un composé R'-OH ou un mélange de composés R'-OH, dans lequel R' est défini comme ci-dessus, en présence d'un ou de plusieurs acides,

**caractérisé en ce que**,
le rapport molaire ions de fer dans les mélanges réactionnels de l'étape (S1) et/ou (S2) sur composé I utilisé dans l'étape (S1) ne dépasse pas 40 ppm.

2. Procédé selon la revendication 1, dans lequel X dans l'étape (S1) indique I.

3. Procédé selon l'une quelconque des revendications 1 et 2, dans lequel du chlorure ou bromure de $C_{3\text{-}4}$-alkyl-magnésium, de préférence du chlorure d'isopropylmagnésium, est utilisé dans l'étape (S1) et en outre du chlorure de lithium est utilisé dans l'étape (S1).

4. Procédé selon l'une quelconque des revendications 1 à 3, dans lequel $R^2$ indique un triméthylsilyle dans le composé de formule générale II utilisé dans l'étape (S2).

5. Procédé selon l'une quelconque des revendications 1 à 4, dans lequel R' dans l'étape (S3) indique un méthyle.

6. Procédé de préparation d'un composé de formule générale IV

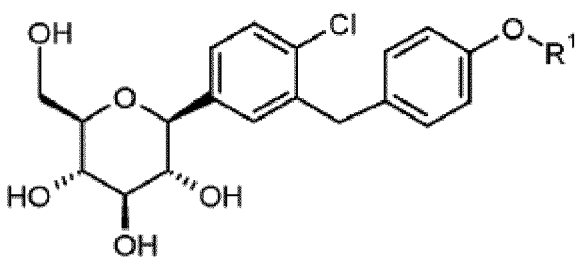

IV

dans lequel $R^1$ est défini comme ci-dessus ; comprenant le procédé selon l'une quelconque des revendications 1 à 5 et en outre comprenant l'étape (S4) et comprenant facultativement l'étape (S5) :

(S4) : la réaction du composé de formule générale III avec un agent réducteur ; et facultativement
(S5) : le clivage des groupements protecteurs $R^2$ qui ne sont pas un hydrogène dans le composé formé à partir du composé de formule générale III dans l'étape (S4).

7. Procédé selon l'une quelconque des revendications 1 à 6, dans lequel, dans le réactif comprenant l'espèce alkyl-magnésium utilisée dans l'étape (S1) et/ou dans une solution comprenant un tel réactif, le rapport molaire ions de fer sur espèces $C_{1\text{-}4}$-alkyl-magnésium ne dépassent pas 40 ppm.

8. Procédé selon l'une quelconque des revendications 1 à 7, dans lequel les matériaux des surfaces d'équipement qui peuvent entrer en contact avec une solution comprenant les espèces alkyl-magnésium utilisées dans l'étape

(S1) et/ou avec les mélanges réactionnels des étapes (S1) et/ou (S2) sont résistants contre la libération ou la lixiviation des ions de fer de sorte que le rapport molaire ions de fer dans les mélanges réactionnels de l'étape (S1) et/ou de l'étape (S2) sur composé I utilisé dans l'étape (S1) ne dépasse pas 40 ppm.

**9.** Procédé selon l'une quelconque des revendications 1 à 8, dans lequel les matériaux des surfaces d'équipement qui peuvent entrer en contact avec une solution comprenant les espèces alkyl-magnésium utilisées dans l'étape (S1) sont résistants contre la libération ou la lixiviation des ions de fer de sorte que, dans ladite solution, le rapport molaire ions de fer sur espèces $C_{1-4}$-alkyl-magnésium ne dépassent pas 40 ppm.

**10.** Procédé selon l'une quelconque des revendications 1 à 9, dans lequel les matériaux des surfaces d'équipement qui peuvent entrer en contact avec une solution comprenant les espèces alkyl-magnésium utilisées à l'étape (S1) et/ou avec les mélanges réactionnels des étapes (S1) et/ou (S2) sont sélectionnés dans le groupe consistant en les alliages métalliques ayant des fractions massiques de fer ne dépassant pas 10 %.

**11.** Procédé selon l'une quelconque des revendications 1 à 10, dans lequel les matériaux des surfaces d'équipement qui peuvent entrer en contact avec une solution comprenant les espèces alkyl-magnésium utilisées dans l'étape (S1) et/ou les mélanges réactionnels des étapes (S1) et/ou (S2) sont sélectionnés dans le groupe consistant en les matériaux qui sont traités et/ou revêtus pour empêcher la libération ou la lixiviation des ions de fer.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005092877 A
- WO 2006117359 A
- WO 2006120208 A
- WO 2011039108 A

### Non-patent literature cited in the description

- **KHARASCH et al.** *J. Am. Chem. Soc.,* 1941, vol. 63, 2316
- **FÜRSTNER et al.** *J. Am. Chem. Soc.,* 2002, vol. 124, 13856